# FASCICULE DE BREVET EUROPEEN

(11) **EP 1 464 327 B1**
(45) Date de publication et mention de la délivrance du brevet: **13.12.2006**
(21) Numéro de dépôt: 04290870.7
(22) Date de dépôt: 01.04.2004
(51) Int. Cl.: A61K 8/34, A61K 8/41, A61K 8/49, A61Q 5/08, A61Q 5/10, A61Q 19/00

(54) **Composition de coloration pour matieres keratiniques humaines comprenant un colorant fluorescent et un polyol, procédé et utilisation**
Zusammensetzung zum Färben von menschlichen Keratinfasern, enthaltend einen Fluoreszenzfarbstoff und ein Polyol, Verfahren und Verwendung
Composition for dyeing human keratinous fibres comprising a fluorescent dyeing agent and a polyol, method and use

(30) Priorité: 01.04.2003 FR 0304030
(43) Date de publication de la demande: 06.10.2004
(73) Titulaire: L'ORÉAL, 75008 Paris (FR)
(72) Inventeur: Plos, Grégory, Yoga-Sekataya-ku 158-0097 (JP); Gourlaouen, Luc, 92600 Asnieres (FR)
(74) Mandataire: Wattremez, Catherine

(56) Documents cités:
- WO-A-99/13841
- WO-A-99/13847
- US-A- 5 445 655
- US-A- 5 830 446

## Description

L'invention concerne une composition comprenant au moins un colorant fluorescent et au moins un polyol particuliers, ainsi que les procédés et dispositif mettant en oeuvre cette composition. Elle concerne également l'utilisation d'une composition comprenant au moins un colorant fluorescent et au moins un polyol particulier, pour colorer avec un effet éclaircissant les matières kératiniques humaines et plus particulièrement les fibres kératiniques comme les cheveux pigmentés ou colorés artificiellement, ou la peau foncée.

Il est fréquent que les personnes ayant une peau foncée désirent s'éclaircir la peau et utilisent dans ce but des compositions cosmétiques ou dermatologiques contenant des agents de blanchiment.
Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.
Toutefois ces agents ne sont pas dépourvus d'inconvénients. En particulier, il est nécessaire de les utiliser de façon prolongée et en des quantités élevées, pour obtenir un effet de blanchiment de la peau. On n'observe pas un effet immédiat à l'application de compositions les comprenant.
En outre, l'hydroquinone et ses dérivés sont utilisés en quantité efficace pour voir apparaître un effet blanchissant. En particulier, l'hydroquinone est connue pour sa cytotoxicité vis-à-vis du mélanocyte.
Par ailleurs, l'acide kojique et ses dérivés présentent l'inconvénient d'être coûteux et de ne pouvoir, pour cette raison, être utilisés en quantité importante dans des produits à large diffusion commerciale.

Il subsiste donc le besoin de compositions cosmétiques permettant d'obtenir un teint plus clair, uniforme, homogène, d'aspect naturel, ces compositions présentant une transparence satisfaisante après application sur la peau.

Dans le domaine capillaire, Il existe principalement deux grands types de coloration capillaire.
Le premier est la coloration semi-permanente ou coloration directe qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. Les colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.
Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants. Il est souvent nécessaire d'associer aux bases d'oxydation et coupleurs, un ou plusieurs colorants directs afin de neutraliser ou de rabattre les nuances trop en reflets rouges, orangés ou dorés, ou au contraire d'accentuer ces reflets rouges, orangés ou dorés.
Parmi les colorants directs disponibles, les colorants directs nitrés benzéniques ne sont pas suffisamment puissants, les indoamines, les colorants quinoniques ainsi que les colorants naturels présentent une faible affinité pour les fibres kératiniques et de ce fait conduisent à des colorations qui ne sont pas assez résistantes vis à vis des différents traitements que peuvent subir les fibres, et en particulier vis à vis des shampooings.

En outre, il existe un besoin d'obtenir un effet d'éclaircissement des fibres kératiniques humaines. Cet éclaircissement est obtenu classiquement par un procédé de décoloration des mélanines du cheveu par un système oxydant, généralement constitué par du peroxyde d'hydrogène associé ou non à des persels. Ce système de décoloration présente l'inconvénient de dégrader les fibres kératiniques et d'altérer leurs propriétés cosmétiques.

La présente invention a pour objet de résoudre les problèmes mentionnés ci-dessus et notamment de proposer une composition qui présente une bonne affinité tinctoriale pour les matières kératiniques et notamment les fibres kératiniques, de bonne propriété de ténacités vis à vis des agents extérieurs, et en particulier vis-à-vis des shampooings, et qui permettent également d'obtenir un éclaircissement sans altération de la matière traitée, plus particulièrement la fibre kératinique.

Il a donc été trouvé de façon inattendue et surprenante que l'utilisation de colorants fluorescents, dans la gamme des orangés, en présence de polyols particuliers, permettait d'atteindre ces objectifs.

La présente invention a donc pour premier objet une composition, comprenant, dans un milieu cosmétiquement acceptable, au moins un colorant fluorescent soluble dans le milieu et au moins un polyol de poids moléculaire inférieur à 500 g/mol et contenant plus de trois atomes de carbone de formule (I) suivante : 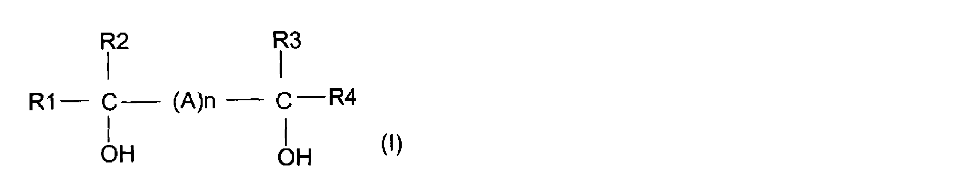 dans laquelle :
A désigne le radical divalent d'une chaîne hydrocarbonée linéaire ou ramifiée, saturée ou insaturée, éventuellement interrompue par 2 à 10 hétéroatomes et comportant de 1 à 35 atomes de carbone.
R1 à R₃ désignent indépendamment l'un de l'autre un atome d'hydrogène, un radical alkyle en C₁-C₂, un radical mono ou polyhydroxyalkyle en C₁-C₆.
n désigne 0 ou 1 ;
la composition ne comprenant pas, à titre d'agent fluorescent, du 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure.

Un second objet de l'invention concerne un procédé pour colorer avec un effet éclaircissant les fibres kératiniques humaines, dans lequel on met en oeuvre les étapes suivantes :
a) on applique sur lesdites fibres une composition selon l'invention, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres.

Un autre objet de l'invention est constitué par un procédé pour colorer avec un effet éclaircissant une peau foncée, dans lequel on applique sur la peau la composition selon l'invention, puis on sèche ou on laisse sécher la peau.

Un autre objet de l'invention concerne l'utilisation pour colorer avec un effet éclaircissant les matières kératiniques humaines, d'une composition comprenant, dans un milieu cosmétiquement acceptable, au moins un colorant fluorescent soluble dans le milieu et au moins un polyol de poids moléculaire inférieur à 500 g/mol et contenant plus de trois atomes de carbone de formule (I) suivante : 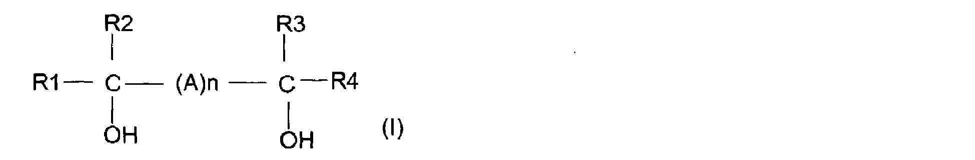 dans laquelle:
A désigne le radical divalent d'une chaîne hydrocarbonée linéaire ou ramifiée, saturée ou insaturée, éventuellement interrompue par 2 à 10 hétéroatomes et comportant de 1 à 35 atomes de carbone.
R₁ à R₃ désignent indépendamment l'un de l'autre un atome d'hydrogène, un radical alkyle en C₁-C₂, un radical mono ou polyhydroxyalkyle en C₁-C₆.
n désigne 0 ou 1.

Les compositions de l'invention permettent en particulier une meilleure fixation du colorant fluorescent dans les matières kératiniques ce qui se traduit par un effet de fluorescence accru et à un effet d'éclaircissement supérieur à celui obtenu avec le colorant fluorescent utilisé seul.
On constate également une meilleure ténacité du résultat vis-à-vis des lavages ou shampooings.

Mais d'autres caractéristiques et avantages de la présente invention apparaîtront plus clairement à la lecture de la description et des exemples qui vont suivre.

A moins d'une indication différente, les bornes des gammes de valeurs qui sont données dans la description, sont incluses dans ces gammes.

Ainsi, comme cela a été indiqué auparavant, la composition selon l'invention comprend, dans un milieu cosmétiquement acceptable, au moins un colorant fluorescent soluble dans le milieu et au moins un polyol de poids moléculaire inférieur à 500 g/mol et contenant plus de trois atomes de carbone de formule (I) suivante : 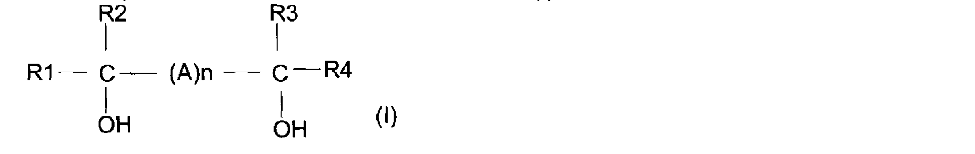 dans laquelle :
A désigne le radical divalent d'une chaîne hydrocarbonée linéaire ou ramifiée, saturée ou insaturée, éventuellement interrompue par 2 à 10 hétéroatomes (plus particulièrement l'oxygène, l'azote) et comportant de 1 à 35 atomes de carbone.
R1 à R₃ désignent indépendamment l'un de l'autre un atome d'hydrogène, un radical alkyle en C₁-C₂, un radical mono ou polyhydroxyalkyle en C₁-C₆.
n désigne 0 ou 1.

De préférence la chaîne hydrocarbonée du radical divalent A de la formule (I) ne comprend pas d'hétéroatomes.

Selon un mode de réalisation particulièrement avantageux de l'invention, le radical A désigne un groupement alkylène linéaire ou ramifié comportant de 1 à 8 atomes de carbone.

A titre de polyols de formule (I) utilisables selon la présente invention, on peut citer sans intention de s'y limiter, le 1,4-butanediol, le 1,5-pentanediol, le 1,6-hexanediol, le néopentylglycol (2,2-diméthyl-1,3-propanediol), l'isoprèneglycol (3-méthyl-1,3-butanediol), le 2,3-diméthyl-1,5-pentanediol, le 2-méthyl-1,3-propanediol, le 3-méthyl-1,5-pentanediol, l'hexylèneglycol (2-méthyl-2,4-pentanediol), le 3-méthyl-1,3,5-pentanetriol et le 1,2,4-butanetriol, seuls ou en mélanges.

Le ou les polyols de formule (I) de la présente invention représentent avantageusement de 0,01 à 30 % en poids, plus particulièrement de 0,1 à 20 % en poids, et de préférence de 0,5 à 10% environ en poids, du poids total de la composition.

La composition comprend en outre au moins un colorant fluorescent.
Par colorant fluorescent, on entend au sens de la présente invention un colorant qui est une molécule qui colore par elle-même, et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éciaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Enfin, le colorant fluorescent mis en oeuvre dans la composition est soluble dans le milieu de la composition. Précisons que le colorant fluorescent est différent en cela d'un pigment fluorescent qui lui, n'est pas soluble dans le milieu de la composition.

Plus particulièrement, le colorant fluorescent utilisé dans le cadre de la présente invention, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

Par ailleurs, selon une caractéristique de l'invention, la composition ne comprend pas, à titre de colorant fluorescent, un 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure.

Conformément à un mode de réalisation encore plus particulier de l'invention, la composition ne comprend pas, à titre de colorant fluorescent, de composé choisi parmi les colorants fluorescents hétérocycliques monocationiques azoiques, azométhiniques ou méthiniques.

Les colorants fluorescents utilisés sont des colorants dans la gamme des orangés.

Les colorants fluorescents de l'invention conduisent à un maximum de réflectance se situant dans la gamme de longueur d'onde allant de 500 à 650 nanomètres, et de préférence dans la gamme de longueur d'onde allant de 550 à 620 nanomètres.

Certains des colorants fluorescents selon la présente invention sont des composés connus en eux-mêmes.

A titre d'exemples de colorants fluorescents susceptibles d'être mis en oeuvre, on peut citer les colorants fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges, de préférence aux familles suivantes : les naphtalimides ; les coumarines cationiques ou non ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

Plus particulièrement, on peut citer parmi eux :
- le Jaune Brilliant B6GL commercialisé par la société SANDOZ et de structure suivante : 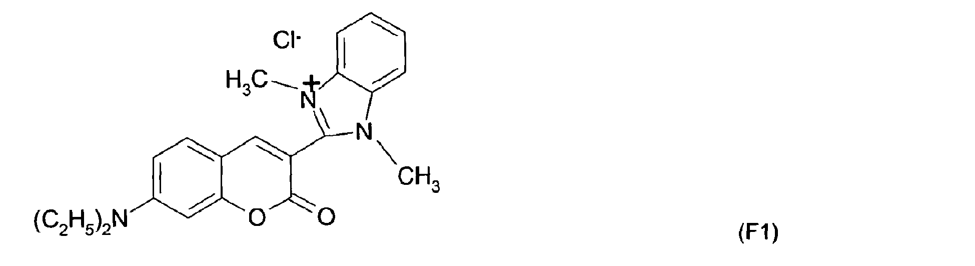
- le Basic Yellow 2, ou Auramine O commercialisé par les sociétés PROLABO, ALDRICH
ou CARLO ERBA et de structure suivante : 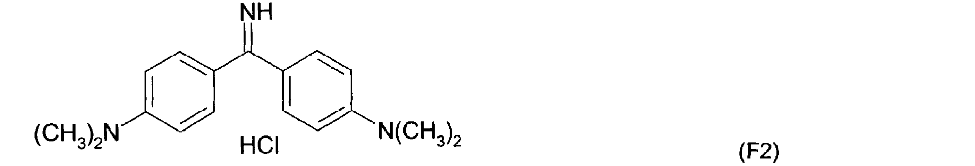 monochlorhydrate de 4,4'-(imidocarbonyl)bis(N,N-diméthylaniline) - CAS number 2465-27-2.

On peut aussi citer les composés de formule suivante : 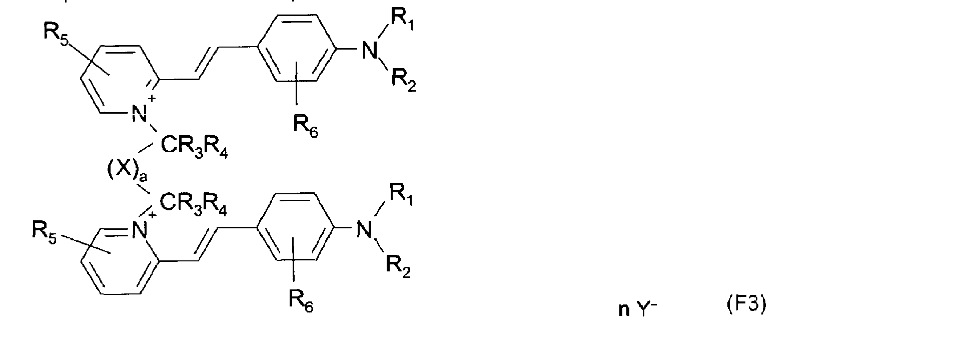 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

Rappelons que les termes hétéroatomes, représentent un atome d'oxygène ou d'azote.
Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).
En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire
ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; -CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.
De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1H-1,2,4-triazolo.

Les radicaux R₁ et R₂, identiques ou différents, peuvent aussi représenter être reliés de manière à former un hétérocycle de formules (I) et (II) suivantes : 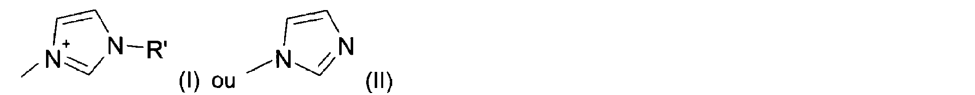 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.
Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.
Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.
De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆₂ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques
ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R₆₇-N(CH₃)₂ ou -R₆₇-N⁺(CH₃)₃ ou -R₆₇-N⁺(CH₂CH₃)₃ avec R₆₇ représentant un radical alkyle en C₁-C₂.
Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.
En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.
Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.
Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).

Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant. Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re-avec Rc représentant un radical alkyle en C₂-C₉, Rd représentant un atome d'hydrogène, un radical alkyle en C₁-C₂ et Re représentant un radical alkyle en C₁-C₆ ; -Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 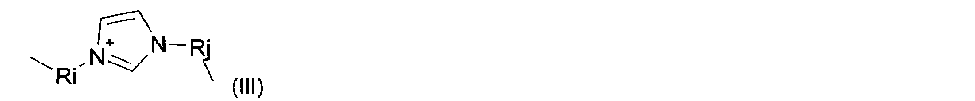
dans laquelle Ri et Rj, identiques ou non, représentent un radical alkyle linéaire en C₁-C₆
X peut de même être choisi parmi les radicaux divalents dérivés de triazine suivants :

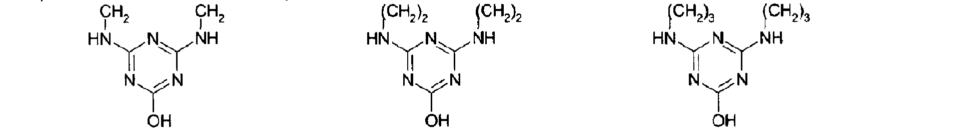

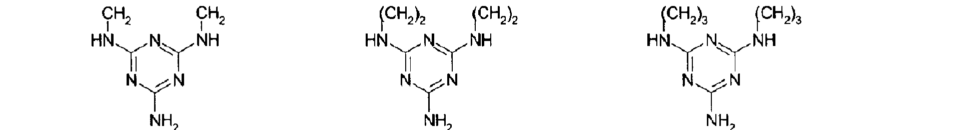

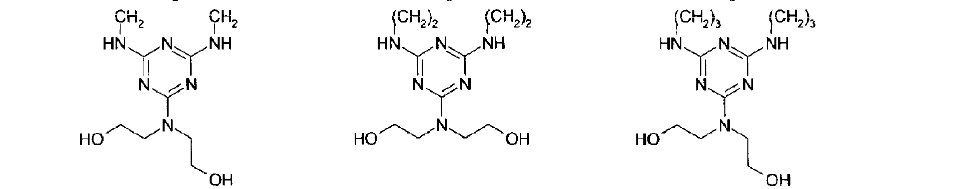

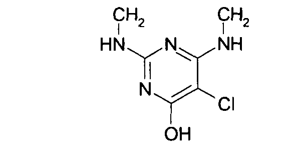

Selon une autre possibilité, X peut représenter les radicaux divalents aromatiques suivants :

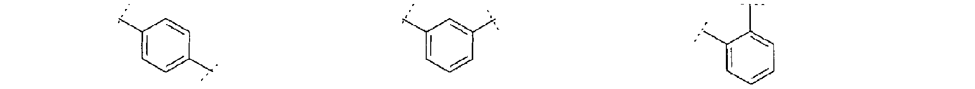

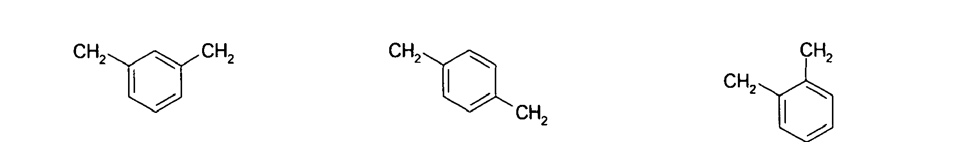

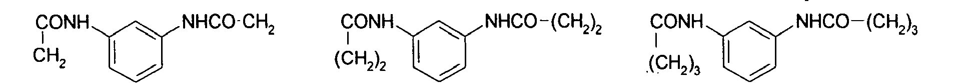

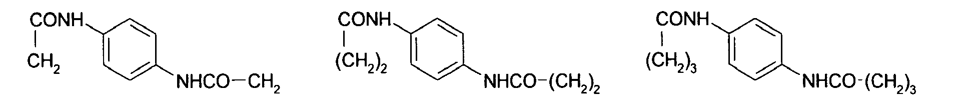

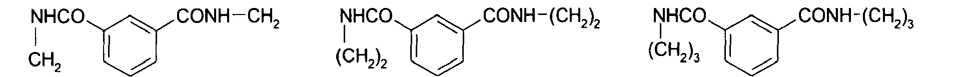

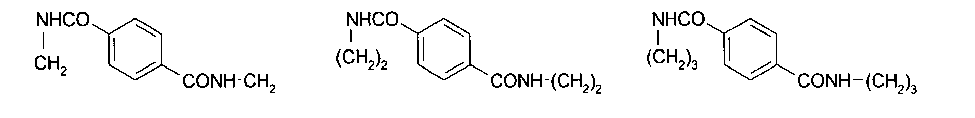

Dans la formule générale de ces composés fluorescents, Y⁻ représente un anion organique ou minéral. S'il y a plusieurs anions Y⁻, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.
Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc. De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore,
ou les groupements de type tolylsulfonyle ou méthylesulfonyle.
Cette première étape peut avoir lieu en présence d'un solvant, bien qu'il ne soit pas obligatoire, comme par exemple le diméthylformamide.
Le nombre de moles d'α-picoline est en général voisin de 2 pour une mole de réactif comprenant les groupes partant.
En outre, la réaction est habituellement mise en oeuvre au reflux du réactif et/ou du solvant s'il est présent.
Le produit issu de cette première étape est ensuite contacté avec un aldéhyde correspondant de formule suivante : 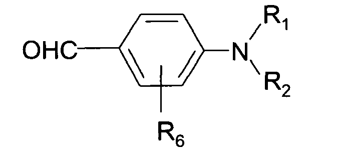 dans laquelle R₁, R₂ et R₆ ont les mêmes significations que précédemment indiquées.
Là encore, la réaction peut être effectuée en présence d'un solvant approprié, de préférence au reflux.
Il est à noter que les radicaux R₁ et R₂ de l'aldéhyde peuvent avoir la signification indiquée dans la formule générale détaillée auparavant.
Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

Le ou les colorants fluorescents présents dans la composition selon l'invention représentent plus particulièrement de 0,01 à 20 % en poids, avantageusement de 0,05 à 10 % en poids et de préférence de 0,1 à 5% en poids, du poids total de la composition.

Le milieu cosmétiquement acceptable est généralement constitué par de l'eau ou par un mélange d'eau et d'un ou plusieurs solvants organiques usuels.

Parmi les solvants convenables, on peut citer plus particulièrement, les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants usuels décrits ci-dessus, s'ils sont présents, représentent habituellement de 1 à 40 % en poids, plus préférentiellement de 5 à 30 % en poids, par rapport au poids total de la composition.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés dans le domaine et notamment en coloration des fibres kératiniques humaines.

Parmi les agents acidifiants, on peut citer, à titre d'exemples, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 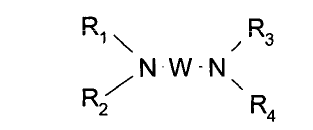 dans laquelle W est un reste propylène éventuellement substitué par un groupement hydroxyle ou un radical alkyle en C₁-C₆ ; R₁, R₂, R₃ et R₄, identiques ou différents, représentent un atome d'hydrogène, un radical alkyle en C₁-C₆ ou hydroxyalkyle en C₁-C₆.

Selon un mode de réalisation particulier de l'invention, la composition peut, comprendre, en plus du ou des colorants fluorescents, un ou plusieurs colorants directs additionnels non fluorescents de nature non ionique, cationique ou anionique, qui peuvent par exemple être choisis parmi les colorants benzéniques nitrés.
Conviennent notamment les colorants directs benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène,
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

La composition conforme à l'invention peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants azoïques, les colorants anthraquinoniques, naphtoquinoniques ou benzoquinoniques, les colorants indigoïdes, ou les colorants dérivés du triarylméthane.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP-A-0 714 954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 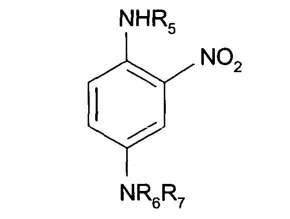
dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans le brevet français FR 2 692 572.

Lorsqu'ils sont présents, le ou les colorants directs additionnels représentent de préférence de 0,0005 à 12 % en poids, et encore plus préférentiellement de 0,005 à 6 % en poids, du poids total de la composition.

Lorsqu'elle est destinée à la coloration d'oxydation, la composition conforme à l'invention comprend, en plus du ou des colorants fluorescents, au moins une base d'oxydation choisie parmi les bases d'oxydation classiquement utilisées pour la coloration d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylènediamine et la 4'aminophényl 1-(3-hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphényiènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylène diamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent plus particulièrement de 0,0005 à 12 % en poids, et de préférence de 0,005 à 6 % en poids, du poids total de la composition.

Lorsqu'elle est destinée à la coloration d'oxydation, la composition conforme à l'invention peut également comprendre, en plus des colorants fluorescents et des bases d'oxydation, au moins un coupleur de façon à modifier ou à enrichir en reflets les nuances obtenues en mettant en oeuvre les colorants fluorescents et la ou les bases d'oxydation.

Les coupleurs utilisables dans la composition conforme à l'invention peuvent être choisis parmi les coupleurs utilisés de façon classique en coloration d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

S'ils sont présents, le ou les coupleurs représentent habituellement de 0,0001 à 10 % en poids, et de préférence de 0,005 à 5 % en poids, du poids total de la composition.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre des compositions de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (A).

La composition conforme à l'invention peut également comprendre divers adjuvants utilisés classiquement, tels que des agents tensio-actifs anioniques, cationiques, non-ioniques, amphotères, zwittérioniques ou leurs mélanges, des polymères anioniques, cationiques, non ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des cations, des polymères cationiques ou amphotères, des silicones volatiles ou non volatiles, modifiées ou non modifiées, des agents filmogènes, des céramides, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

Lorsqu'un ou plusieurs agents tensioactifs sont présents, de préférence de type non ionique, anionique ou encore amphotère, leur teneur représente de 0,01 à 30 % en poids par rapport au poids de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Selon un mode de réalisation préféré de l'invention, la composition selon l'invention se trouve sous la forme d'un shampooing colorant et éclaircissant.

Dans la composition selon l'invention, lorsqu'une ou plusieurs bases d'oxydation sont utilisées, éventuellement en présence d'un ou plusieurs coupleurs, ou lorsque le ou les colorants fluorescents sont utilisés dans le cadre d'une coloration directe éclaircissante, alors la composition conforme à l'invention peut en outre renfermer au moins un agent oxydant choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

L'invention a également pour objet l'utilisation pour colorer avec un effet éclaircissant des matières kératiniques humaines, d'une composition comprenant dans un milieu cosmétiquement acceptable, au moins un colorant fluorescent soluble dans le milieu et au moins un polyol de poids moléculaire inférieur à 500 g/mol et contenant plus de trois atomes de carbone de formule (I) suivante : 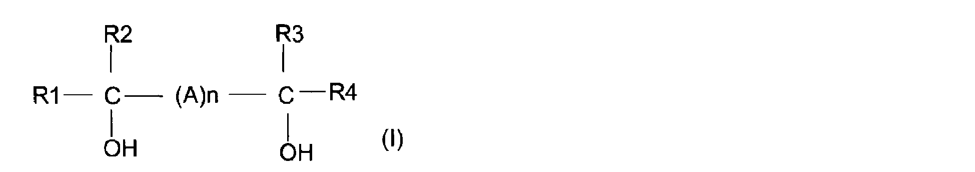 dans laquelle :
A désigne le radical divalent d'une chaîne hydrocarbonée linéaire ou ramifiée, saturée ou insaturée, éventuellement interrompue par 2 à 10 hétéroatomes et comportant de 1 à 35 atomes de carbone.
R1 à R₃ désignent indépendamment l'un de l'autre un atome d'hydrogène, un radical alkyle en C₁-C₂, un radical mono ou polyhydroxyalkyle en C₁-C₆.
n désigne 0 ou 1.

Dans le cadre de cette utilisation, le composé fluorescent peut être choisi parmi les composés fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents monocationiques et polycationiques de type azoïque, azométhinique, ou méthinique, seuls ou en mélanges.

A titre de composés plus particuliers, on peut citer les composés de formules F1, F2 et F3 déjà détaillées auparavant.

On peut de même utiliser les composés de structure (F4) suivante : 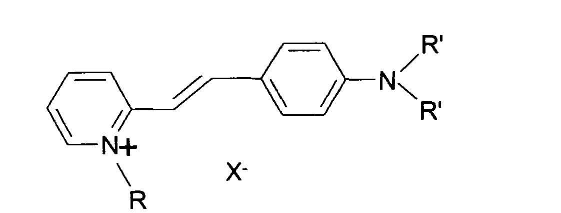 formule dans laquelle R représente un radical méthyle ou éthyle; R' représente un radical méthyle, X- un anion du type chlorure, iodure, sulfate, méthosulfate, acétate, perchlorate. A titre d'exemple de composé de ce type on peut citer le Photosensitiving Dye NK-557 commercialisé par la société UBICHEM, pour lequel R représente un radical éthyle, R' un radical méthyle et X- un iodure.

Tout ce qui a été décrit auparavant sur les natures et teneurs des divers additifs présents dans la composition reste valable et ne sera pas repris dans cette partie.

Selon la présente invention, on entend par matières kératiniques humaines, la peau, les cheveux, les ongles, les cils, et les sourcils, et plus particulièrement la peau foncée et les cheveux pigmentés ou colorés artificiellement.

Au sens de l'invention, on entend par peau foncée, une peau dont la luminance L* chiffrée dans le système C.I.E.L. L*a*b* est inférieure ou égale à 45 et de préférence inférieure ou égale à 40, sachant par ailleurs que L*=0 équivaut au noir et L*=100 au blanc. Les types de peau correspondant à cette luminance sont la peau africaine, la peau afro-américaine, la peau hispano-américaine, la peau indienne et la peau maghrébine.
Au sens de l'invention, on entend par cheveux pigmentés ou colorés artificiellement, des cheveux dont la hauteur de ton est inférieure ou égale à 6 (blond foncé) et de préférence inférieure ou égale à 4 (châtain).
L'éclaircissement des cheveux est évalué par la 'hauteur de ton" qui caractérise le degré ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278.
Les hauteurs de ton s'échelonnent de 1 (noir) à 10 (blond clair), une unité correspondant à un ton ; plus le chiffre est élevé et plus la nuance est claire.

Un autre objet de la présente invention concerne donc un procédé de coloration avec effet éclaircissant de fibres kératiniques humaines consistant à mettre en oeuvre les étapes suivantes :
a) on applique sur les fibres kératiniques, pendant un temps suffisant pour développer la coloration et l'éclaircissement désirés, la composition selon l'invention,
b) on rince éventuellement lesdites fibres,
c) éventuellement on lave au shampooing et on rince lesdites fibres,
d) on sèche ou on laisse sécher les fibres.

La présente invention a en outre pour objet un procédé pour colorer avec un effet éclaircissant une peau foncée dans lequel on applique sur la peau, la composition qui vient d'être décrite, puis à sécher ou laisser sécher la peau.

Tout ce qui a été précédemment décrit concernant les divers éléments constitutifs de la composition reste valable et l'on pourra s'y reporter.

Notamment, les procédés selon l'invention sont appropriés pour traiter des fibres kératiniques humaines, et notamment les cheveux, pigmentées ou colorées artificiellement, ou encore de la peau foncée.
Plus particulièrement, les fibres qui peuvent être avantageusement traitées par le procédé selon l'invention, présentent une hauteur de ton est inférieure ou égale à 6 (blond foncé) et de préférence inférieure ou égale à 4 (châtain).
De plus, une peau foncée susceptible d'être traitée conformément à l'invention, présente une luminance L*, chiffrée dans le système C.I.E.L. L*a*b*, inférieure ou égale à 45 et de préférence inférieure ou égale à 40.

Selon un premier mode de réalisation de l'invention, le procédé de coloration avec effet éclaircissant des fibres est mis en oeuvre avec une composition ne comprenant ni de colorants d'oxydation ni de coupleur et en l'absence d'agent oxydant.

Selon un deuxième mode de réalisation de l'invention, le procédé de coloration avec effet éclaircissant des fibres est mis en oeuvre avec une composition ne comprenant pas de colorants d'oxydation ni de coupleur, mais en présence d'agent(s) oxydant(s).

Selon une première variante de ces procédés de coloration conformes à l'invention, on applique sur les fibres, et notamment les cheveux, au moins une composition telle que définie précédemment, pendant un temps suffisant pour développer la coloration et l'éclaircissement désirés, après quoi on rince, on lave éventuellement au shampooing, on rince à nouveau et on sèche.

Selon une deuxième variante de ces procédés de coloration conformes à l'invention, on applique sur les fibres, et notamment les cheveux au moins une composition telle que définie précédemment, pendant un temps suffisant pour développer la coloration et l'éclaircissement désirés, sans rinçage final.

Selon une troisième variante de procédé de coloration conforme à l'invention, le procédé de coloration comporte une étape préliminaire consistant à stocker sous forme séparée, d'une part, une composition selon l'invention comprenant, outre le colorant fluorescent et le polyol particulier, éventuellement au moins une base d'oxydation et/ou coupleur et, d'autre part, une composition comprenant, dans un milieu cosmétiquement acceptable, au moins un agent oxydant, puis à procéder à leur mélange au moment de l'emploi avant d'appliquer ce mélange sur les fibres kératiniques, et notamment les cheveux, pendant un temps suffisant pour développer la coloration désirée, après quoi on rince les fibres, on les lave éventuellement au shampooing, on les rince à nouveau et on les sèche.

Le temps nécessaire au développement de la coloration et à l'obtention de l'effet éclaircissant sur les fibres, notamment les cheveux, est d'environ 5 à 60 minutes et plus particulièrement d'environ 5 à 40 minutes.

La température nécessaire au développement de la coloration et à l'obtention de l'effet éclaircissant est généralement comprise entre la température ambiante (15 à 25°C) et 80°C et plus particulièrement entre 15 et 40 °C.

Un autre objet de l'invention est un dispositif à plusieurs compartiments pour la coloration avec effet éciaircissant des fibres kératiniques et notamment des cheveux, comprenant au moins un compartiment renfermant une composition selon l'invention, et au moins un autre compartiment renfermant une composition comprenant au moins un agent oxydant. Ce dispositif peut être équipé d'un moyen permettant de délivrer sur les fibres le mélange souhaité, tel que les dispositifs décrits dans le brevet FR 2 586 913.

Il est à noter que la composition selon l'invention, si elle est utilisée pour traiter des fibres kératiniques, telles que des cheveux châtains par exemple, permet d'atteindre les résultats suivants :
Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueur d'onde allant de 500 à 700 nanomètres, est supérieure à celle correspondant aux cheveux non traités.
Cela signifie que, dans la gamme de longueur d'onde allant de 500 à 700 nanomètres, de préférence de 540 à 700 nénomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0, 1 %.
Il est précisé toutefois qu'il peut exister dans la gamme de longueur d'onde allant de 500 à 700 nanomètres, de préférence de 540 à 700 nanomètres, une ou plusieurs plages où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

En outre, et de préférence, la composition selon l'invention est susceptible d'éclaircir les cheveux et la peau dans une nuance qui, chiffrée dans le système C.I.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des fibres kératiniques châtain, plus particulièrement des cheveux, à raison de 10 grammes de composition pour 1 gramme de fibres châtain. La composition est étalée de façon à recouvrir l'ensemble des fibres. On laisse la composition agir pendant 20 minutes à température ambiante (20 à 25°C). Les fibres sont ensuite rincées à l'eau puis lavées avec un shampooing à base de lauryléther sulfate. Elles sont ensuite séchées. On mesure alors les caractéristiques spectrocolorimétriques des fibres pour en déterminer les coordonnées L*a*b*.
Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et -b* est bleu) ; des valeurs proches de zéro pour a* et b* correspondent à des nuances grises.

Les exemples qui suivent sont destinés à illustrer l'invention sans pour autant en limiter la portée.

### EXEMPLES

### Composé fluorescent

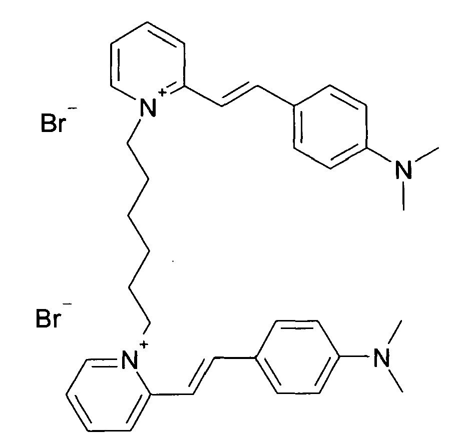

On fait réagir 93 g de 2-picoline avec 120g de 1,6 dibromohexane dans le diméthylformamide à 110°C pendant 5 heures.
On récupère le produit précipité, et on le filtre.
On solubilise 109 g du produit obtenu précédemment dans du méthanol et l'on ajoute 82,82 g de p-diméthylaminobenzaldéhyde en deux fois, en présence de pyrrolidine.
On laisse ensuite pendant 30 minutes.
On récupère le produit sous forme précipitée.

Analyse par spectroscopie de masse : 266.
Analyse élémentaire : C : 62,43 % ; H : 6,40 % ; Br : 23,07 % ; N : 8,09 %.
La formule est la suivante C₃₆H₄₄N₄.2Br.

### Compositions

| **Composition** | **1** | **2** | **3** |
|---|---|---|---|
| Composé fluorescent | 1 % | 1 % | 1 % |
| Hexylèneglycol | 7% | 7% | 7% |
| Eau distillée | qsp. 100% | qsp. 100% | |

### Coloration

La composition est appliquée sur une mèche de cheveux châtains naturels (hauteur de ton 4) avec un temps de pose de 20 minutes.
Les mèches sont ensuite rincées et un séchées au casque pendant 30 minutes.

On obtient un bon effet d'éclaircissement des mèches.

## Revendications

1. Composition **caractérisée par en ce qu**'elle comprend, dans un milieu cosmétiquement acceptable, au moins un colorant fluorescent dans la gamme des orangés, soluble dans le milieu, et au moins un polyol de poids moléculaire inférieur à 500 g/mol et contenant plus de trois atomes de carbone, de formule (I) suivante :
dans laquelle: 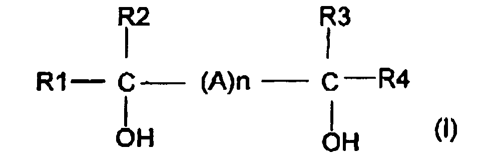
A désigne le radical divalent d'une chaîne hydrocarbonée linéaire ou ramifiée, saturée ou insaturée, éventuellement interrompue par 2 à 10 hétéroatomes et comportant de 1 à 35 atomes de carbone ;
R₁ à R₄ désignent indépendamment l'un de l'autre un atome d'hydrogène, un radical alkyle en C₁-C₂, un radical mono ou polyhydroxyalkyle en C₁-C₆;
n désigne 0 ou 1 ;
la composition ne comprenant pas, à titre d'agent fluorescent, du 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure.

2. Composition selon la revendication 1, **caractérisée en ce que** la chaîne hydrocarbonée du radical divalent A ne comprend pas d'hétéroatome.

3. Composition selon l'une des revendications 1 ou 2, **caractérisée en ce que** le radical A désigne un groupement alkylène linéaire ou ramifié, comportant de 1 à 8 atomes de carbone.

4. Composition selon l'une quelconque des revendications 1 à 3, **caractérisée par le fait que** les polyols de formule (I) sont choisis parmi le 1,4-butanediol, le 1,5-pentanediol, le 1,6-hexanediol, le néopentylglycol, l'isoprèneglycol, l'hexylèneglycol, le 2,3-diméthyl-1,5-pentanediol, le 2-méthyl-1,3-propanediol, le 3-méthyl-1,5-pentanedlol, le 3-méthyl-1,3,5-pentanetriol et le 1,2,4-butanetriol, seuls ou en mélanges.

5. Composition selon l'une quelconque des revendications 1 à 4, **caractérisée en ce que** le ou les polyols de formule (I) représentent de 0,01 à 30 % en poids, plus préférentiellement de 0,1 à 20 % en poids et plus particulièrement encore de 0,5 à 10% en poids, du poids total de la composition.

6. Composition selon l'une quelconque des revendications précédentes, **caractérisée en ce que** le colorant fluorescent, éventuellement neutralisé, est soluble dans le milieu cosmétiquement acceptable à au moins 0,001 g/l, plus particulièrement au moins 0,5 g/l, de préférence au moins 1 g/l et selon un mode de réalisation encore plus préféré, au moins 5 g/l, à la température comprise entre 15 et 25°C.

7. Composition selon l'une quelconque des revendications précédentes, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

8. Composition selon l'une quelconque des revendications précédentes, **caractérisée en ce que** le colorant fluorescent est choisi parmi parmi les colorants fluorescents appartenant aux familles suivantes : les naphtalimides ; les coumarines cationiques ou non ; les xanthénodiquinolizines ; les azaxanthènes ; les naphtolactames : les azlactones ; les oxazines ; les thiazines ; les dioxazines ; les colorants fluorescents polycationiques de type azoïque, azométhinique, ou méthinique, seuls ou en mélanges.

9. Composition selon l'une quelconque des revendications précédentes, **caractérisée en ce que** le colorant fluorescent est choisi dans le groupe formé par les colorants de structures suivantes : 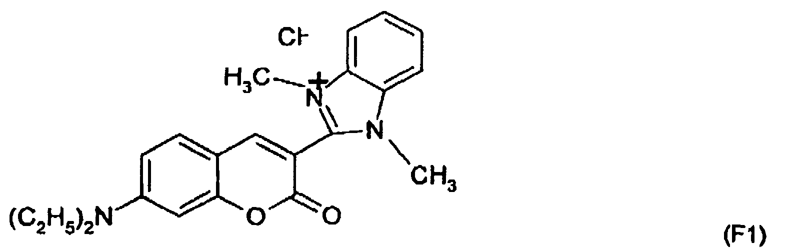 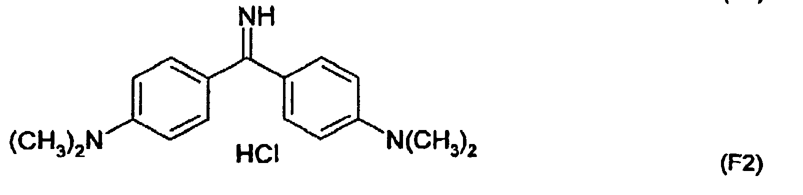 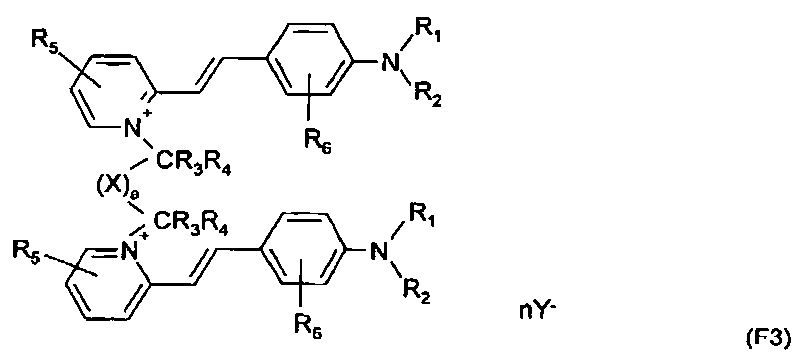 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral;
n étant un nombre entier au moins égal à 2 et au plus égal au nombre de charges cationiques présentes dans le composé fluorescent.

10. Composition selon l'une quelconque des revendications précédentes, **caractérisée en ce que** le ou les colorants fluorescents sont présents dans une concentration pondérale allant de 0,01 à 20% en poids, plus particulièrement de 0,05 à 10% en poids, de préférence de 0,1 à 5% en poids, par rapport au poids total de la composition.

11. Composition selon l'une quelconque des revendications précédentes, **caractérisée en ce qu**'elle comprend en outre au moins un colorant direct additionnel non fluorescent de nature non ionique, cationique ou anionique.

12. Composition selon la revendication 11, **caractérisée par le fait que** les colorants directs additionnels non fluorescents sont choisis parmi les colorants benzéniques nitrés, les colorants azoïques, les colorants anthraquinoniques, naphtoquinoniques ou benzoquinoniques, les colorants indigoïdes, les colorants dérivés du triarylméthane, ou leurs mélanges.

13. Composition selon l'une quelconque des revendications 11 ou 12, **caractérisée par le fait que** le ou les colorants directs additionnels représentent de 0,0005 à 12 % en poids, plus particulièrement de 0,005 à 6 % en poids, du poids total de la composition.

14. Composition selon l'une quelconque des revendications précédentes, **caractérisée en ce qu'**elle se présente sous la forme d'un shampooing éclaircissant et colorant.

15. Composition selon l'une quelconque des revendications précédentes, **caractérisée en ce qu'**elle comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcatin.

16. Composition selon la revendication 15, **caractérisée en ce que** la ou les bases d'oxydation représentent 0,0005 à 12 % en poids, plus particulièrement 0,005 à 6 % en poids, du poids total de la composition.

17. Composition selon l'une quelconque des revendications 15 ou 16, **caractérisée en ce qu**'elle comprend au moins un coupleur choisi parmi les métaphénylène diamines, les méta-aminophénols, les métadiphénols et les coupleurs hétérocycliques ou leurs sels d'addition avec un acide ou avec un agent alcalin.

18. Composition selon la revendication 17, **caractérisée en ce que** le ou les coupleurs représentent de 0,0001 à 10 % en poids, plus particulièrement de 0,005 à 5 % en poids, du poids total de la composition tinctoriale.

19. Composition selon l'une quelconque des revendications précédentes, **caractérisée en ce qu**'elle renferme au moins un agent oxydant.

20. Composition selon la revendication 19, **caractérisée en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons.

21. Procédé pour colorer avec effet éclaircissant des cheveux présentant une hauteur de ton inférieure ou égale à 6, mettant en oeuvre, une composition comprenant dans un milieu cosmétiquement acceptable, au moins un colorant fluorescent soluble dans le milieu, et au moins un polyol de poids moléculaire inférieur à 500 g/mol et contenant plus de trois atomes de carbone, de formule (I) suivante :
dans laquelle : 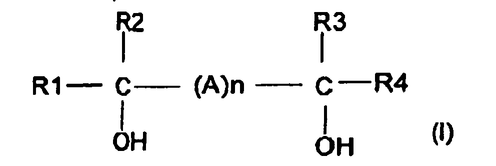
A désigne le radical divalent d'une chaîne hydrocarbonée linéaire ou ramifiée, saturée ou insaturée, éventuellement interrompue par 2 à 10 hétéroatomes et comportant de 1 à 35 atomes de carbone ;
R₁ à R₄ désignent indépendamment l'un de l'autre un atome d'hydrogène, un radical alkyle en C₁-C₂, un radical mono ou polyhydroxyalkyle en C₁-C₆ ;
n désigne 0 ou 1.

22. Procédé selon la revendication 21, **caractérisé en ce que** le colorant fluorescent est un colorant dans la gamme des orangés.

23. Procédé selon l'une quelconque des revendications 21 ou 22, **caractérisé en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

24. Procédé selon l'une quelconque des revendications 21 à 23 **caractérisé en ce que** le colorant fluorescent est choisi parmi les composés fluorescents appartenant aux familles suivantes : les naphtalimides ; les coumarines cationiques ou non ; les xanthénodiquinolizines (comme notamment les sulforhodamines) : les azaxanthènes ; les naphtolactames : les azlactones : les oxazines ; les thiazines ; les dioxazines ; les colorants fluorescents monocationiques et polycationiques de type azoïque, azométhinique, ou méthinique, seuls ou en mélanges.

25. Procédé selon l'une quelconque des revendications 21 à 24, **caractérisé en ce que** le colorant fluorescent est choisi dans le groupe formé par les colorants de structures suivantes : 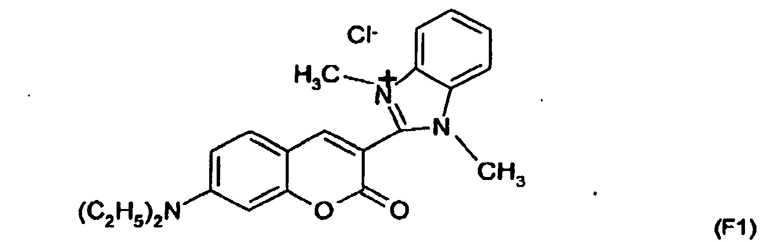 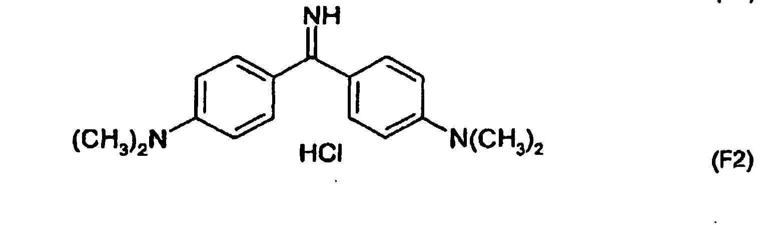 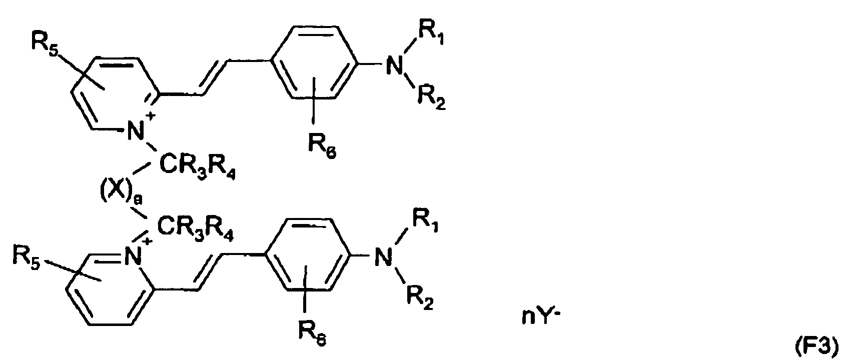 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène : un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent; 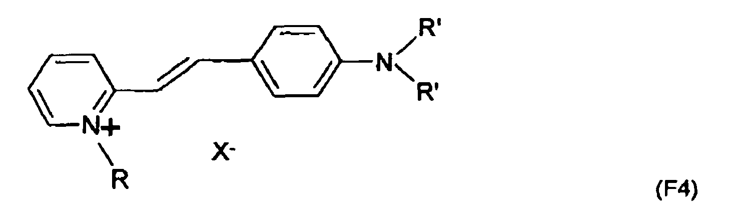
formule dans laquelle R représente un radical méthyle ou éthyle; R' représente un radical méthyle, X- un anion du type chlorure, iodure, sulfate, méthosulfate, acétate, perchlorate.

26. Procédé selon la revendication 21 à 25, **caractérisé par le fait que** les cheveux sont pigmentés ou colorés artificiellement.

27. Procédé selon l'une quelconque des revendications 21 à 26, **caractérisé par le fait que** les cheveux présentent une hauteur de ton inférieure ou égale à 4.

28. Procédé de coloration avec effet éclaircissant selon l'une quelconque des revendications 21 à 27, consistant à mettre en oeuvre les étapes suivantes :
a) on applique sur les fibres kératiniques, pendant un temps suffisant pour développer la coloration et l'éclaircissement désirés, la composition telle que définie dans l'une quelconque des revendications 21 a 25,
b) on rince éventuellement lesdites fibres,
c) éventuellement on lave au shampooing et on rince lesdites fibres.
d) on sèche ou on laisse sécher les fibres.

29. Procédé selon la revendication 28, **caractérisé en ce qu**'il comporte une étape préliminaire consistant à stocker sous forme séparée, d'une part, une composition telle que définie dans l'une des revendications à 21 à 25 et d'autre part, une composition renfermant, dans un milieu cosmétiquement acceptable, au moins un agent oxydant, puis à procéder à leur mélange au moment de l'emploi avant d'appliquer ce mélange sur les fibres pendant un temps suffisant pour développer la coloration désirée, après quoi on rince, on lave éventuellement au shampooing, on rince à nouveau et on sèche.

30. Procédé pour colorer avec un effet éclaircissant une peau foncée, **caractérisé en ce que** l'on applique sur la peau une composition selon l'une quelconque des revendications 1 à 13, puis à sécher ou laisser sécher la peau.

31. Dispositif à plusieurs compartiments pour la coloration et l'éclaircissement des fibres kératiniques humaines, comprenant au moins un compartiment renfermant une composition selon l'une des revendications 1 à 13 et 15 à 18 et au moins un autre compartiment renfermant une composition renfermant au moins un agent oxydant.

## Claims

1. Composition, **characterized in that** it comprises, in a cosmetically acceptable medium, at least one dye fluorescent in the orange range that is soluble in the medium and at least one polyol with a molecular weight of less than 500 g/mol and containing more than three carbon atoms, of formula (I) below: 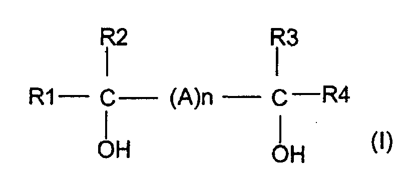 in which:
A denotes the divalent radical of a linear or branched, saturated or unsaturated hydrocarbon-based chain, optionally interrupted with 2 to 10 hetero atoms and containing from 1 to 35 carbon atoms,
R₁ to R₄ denote, independently of each other, a hydrogen atom, a C₁-C₂ alkyl radical or a C₁-C₆ mono- or polyhydroxyalkyl radical,
n denotes 0 or 1;
the composition not comprising, as fluorescent agent, 2-[2-(4-dialkylamino)phenylethenyl]-1-alkylpyridinium in which the alkyl radical of the pyridinium nucleus represents a methyl or ethyl radical, that of the benzene nucleus represents a methyl radical and in which the counterion is a halide.

2. Composition according to Claim 1, **characterized in that** the hydrocarbon-based chain of the divalent radical A does not comprise any hetero atoms.

3. Composition according to either of Claims 1 and 2, **characterized in that** the radical A denotes a linear or branched alkylene group containing from 1 to 8 carbon atoms.

4. Composition according to any one of Claims 1 to 3, **characterized in that** the polyols of formula (I) are chosen from 1,4-butanediol, 1,5-pentanediol, 1,6-hexanediol, neopentyl glycol, isoprene glycol, hexylene glycol, 2,3-dimethyl-1,5-pentanediol, 2-methyl-1,3-propanediol, 3-methyl-1,5-pentanediol, 3-methyl-1,3,5-pentanetriol and 1,2,4-butanetriol, alone or as mixtures.

5. Composition according to any one of Claims 1 to 4, **characterized in that** the polyol(s) of formula (I) represent(s) from 0.01% to 30% by weight, more preferably from 0.1% to 20% by weight and even more particularly from 0.5% to 10% by weight, relative to the total weight of the composition.

6. Composition according to any one of the preceding claims, **characterized in that** the optionally neutralized fluorescent dye is soluble in the cosmetically acceptable medium to at least 0.001 g/l, more particularly at least 0.5 g/l and preferably at least 1 g/l, and according to an even more preferred embodiment at least 5 g/l, at a temperature of between 15 and 25°C.

7. Composition according to any one of the preceding claims, **characterized in that** the fluorescent dye leads to a reflectance maximum that is in the wavelength range from 500 to 650 nanometres and preferably in the wavelength range from 550 to 620 nanometres.

8. Composition according to any one of the preceding claims, **characterized in that** the fluorescent dye is chosen from the fluorescent dyes belonging to the following families: naphthalimides; cationic or non-cationic coumarins; xanthenodiquinolizines; azaxanthenes; naphtholactams; azlactones; oxazines; thiazines; dioxazines; polycationic fluorescent dyes of azo, azomethine or methine type, alone or as mixtures.

9. Composition according to any one of the preceding claims, **characterized in that** the fluorescent dye is chosen from the group formed by the dyes having the following structures: 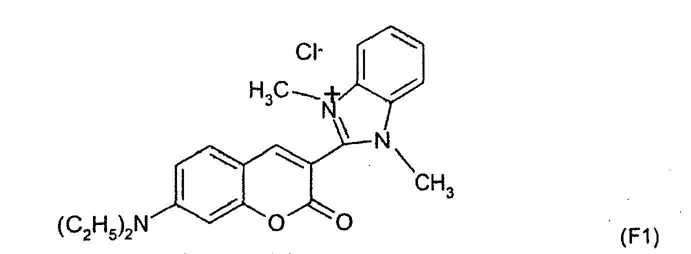 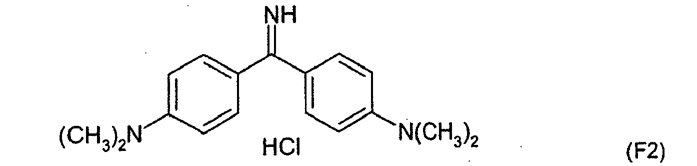 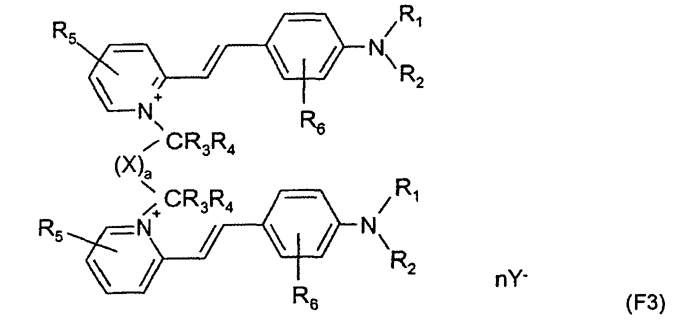 in which:
R₁ and R₂, which may be identical or different, represent:
• a hydrogen atom;
• a linear or branched alkyl radical containing 1 to 10 carbon atoms and preferably from 1 to 4 carbon atoms, optionally interrupted and/or substituted with at least one hetero atom and/or group comprising at least one hetero atom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one hetero atom and/or group comprising at least one hetero atom and/or substituted with at least one halogen atom;
• R₁ and R₂ may optionally be linked so as to form a heterocycle with the nitrogen atom and may comprise one or more other hetero atoms, the heterocycle optionally being substituted with at least one linear or branched alkyl radical preferably containing from 1 to 4 carbon atoms and optionally being interrupted and/or substituted with at least one hetero atom and/or group comprising at least one hetero atom and/or substituted with at least one halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one hetero atom;
R₆, which may be identical or different, represent a hydrogen atom; a halogen atom; a linear or branched alkyl radical containing 1 to 4 carbon atoms, optionally substituted and/or interrupted with at least one hetero atom and/or group bearing at least one hetero atom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one hetero atom and/or group containing at least one hetero atom and/or substituted with at least one halogen atom;
• a 5- or 6-membered heterocyclic radical optionally substituted with at least one linear or branched alkyl radical containing 1 to 14 carbon atoms, optionally substituted with at least one hetero atom; with at least one linear or branched aminoalkyl radical containing 1 to 4 carbon atoms, optionally substituted with at least one hetero atom; with at least one halogen atom;
• a fused or non-fused aromatic or diaromatic radical, optionally separated with an alkyl radical containing 1 to 4 carbon atoms, the aryl radical(s) optionally being substituted with at least one halogen atom or with at least one alkyl radical containing 1 to 10 carbon atoms optionally substituted and/or interrupted with at least one hetero atom and/or group bearing at least one hetero atom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound.

10. Composition according to any one of the preceding claims, **characterized in that** the fluorescent dye(s) is(are) present in a weight concentration ranging from 0.01% to 20% by weight, more particularly from 0.05% to 10% by weight and preferably from 0.1% to 5% by weight relative to the total weight of the composition.

11. Composition according to any one of the preceding claims, **characterized in that** it also comprises at least one additional non-fluorescent direct dye of nonionic, cationic or anionic nature.

12. Composition according to Claim 11, **characterized in that** the additional non-fluorescent direct dyes are chosen from nitrobenzene dyes, azo dyes, anthraquinone dyes, naphthoquinone dyes, benzoquinone dyes, indigoid dyes, triarylmethane-based dyes, or mixtures thereof.

13. Composition according to either of Claims 11 and 12, **characterized in that** the additional direct dye(s) represent (s) from 0.0005% to 12% by weight and more particularly from 0.005% to 6% by weight relative to the total weight of the composition.

14. Composition according to any one of the preceding claims, **characterized in that** it is in the form of a lightening dyeing shampoo.

15. Composition according to any one of the preceding claims, **characterized in that** it comprises at least one oxidation base chosen from para-phenylenediamines, bis(phenyl)alkylenediamines, para-aminophenols, ortho-aminophenols and heterocyclic bases, or the addition salts thereof with an acid or with an alkaline agent.

16. Composition according to Claim 15, **characterized in that** the oxidation base(s) represent(s) 0.0005% to 12% by weight and more particularly 0.005% to 6% by weight relative to the total weight of the composition.

17. Composition according to either of Claims 15 and 16, **characterized in that** it comprises at least one coupler chosen from meta-phenylenediamines, meta-aminophenols, meta-diphenols and heterocyclic couplers, or the addition salts thereof with an acid or with an alkaline agent.

18. Composition according to Claim 17, **characterized in that** the coupler(s) represent(s) from 0.0001% to 10% by weight and more particularly from 0.005% to 5% by weight relative to the total weight of the dye composition.

19. Composition according to any one of the preceding claims, **characterized in that** it contains at least one oxidizing agent.

20. Composition according to Claim 19, **characterized in that** the oxidizing agent is chosen from hydrogen peroxide, urea peroxide, alkali metal bromates, persalts such as perborates and persulphates, and enzymes such as peroxidases and two-electron or four-electron oxidoreductases.

21. Process for dyeing with a lightening effect hair having a tone height of less than or equal to 6, using a composition comprising, in a cosmetically acceptable medium, at least one fluorescent dye that is soluble in the medium and at least one polyol with a molecular weight of less than 500 g/mol and containing more than three carbon atoms, of formula (I) below: 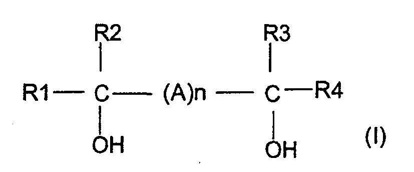 in which:
A denotes the divalent radical of a linear or branched, saturated or unsaturated hydrocarbon-based chain, optionally interrupted with 2 to 10 hetero atoms and containing from 1 to 35 carbon atoms,
R₁ to R₄ denote, independently of each other, a hydrogen atom, a C₁-C₂ alkyl radical or a C₁-C₆ mono- or polyhydroxyalkyl radical,
n denotes 0 or 1.

22. Process according to Claim 21, **characterized in that** the fluorescent dye is a dye in the orange range.

23. Process according to either of Claims 21 and 22, **characterized in that** the fluorescent dye leads to a reflectance maximum that is in the wavelength range from 500 to 650 nanometres and preferably in the wavelength range from 550 to 620 nanometres.

24. Process according to any one of Claims 21 to 23, **characterized in that** the fluorescent dye is chosen from the fluorescent compounds belonging to the following families: naphthalimides; cationic or non-cationic coumarins; xanthenodiquinolizines (especially such as sulphorhodamines); azaxanthenes; naphtholactams; azlactones; oxazines; thiazines; dioxazines; monocationic and polycationic fluorescent dyes of azo, azomethine or methine type, alone or as mixtures.

25. Process according to any one of Claims 21 to 24, **characterized in that** the fluorescent dye is chosen from the group formed by dyes having the following structures: 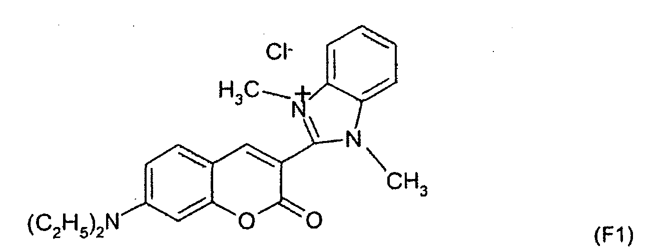 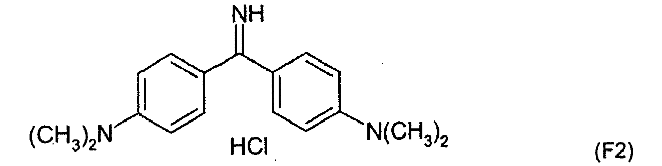 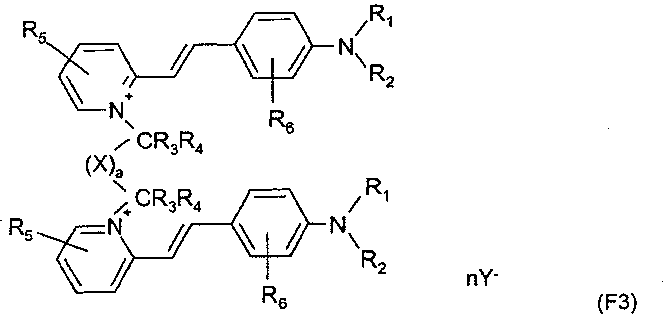 in which:
R₁ and R₂, which may be identical or different, represent:
• a hydrogen atom;
• a linear or branched alkyl radical containing 1 to 10 carbon atoms and preferably from 1 to 4 carbon atoms, optionally interrupted and/or substituted with at least one hetero atom and/or group comprising at least one hetero atom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one hetero atom and/or group comprising at least one hetero atom and/or substituted with at least one halogen atom;
• R₁ and R₂ may optionally be linked so as to form a heterocycle with the nitrogen atom and may comprise one or more other hetero atoms, the heterocycle optionally being substituted with at least one linear or branched alkyl radical preferably containing from 1 to 4 carbon atoms and optionally being interrupted and/or substituted with at least one hetero atom and/or group comprising at least one hetero atom and/or substituted with at least one halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one hetero atom;
R₆, which may be identical or different, represent a hydrogen atom; a halogen atom; a linear or branched alkyl radical containing 1 to 4 carbon atoms, optionally substituted and/or interrupted with at least one hetero atom and/or group bearing at least one hetero atom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one hetero atom and/or group containing at least one hetero atom and/or substituted with at least one halogen atom;
• a 5- or 6-membered heterocyclic radical optionally substituted with at least one linear or branched alkyl radical containing 1 to 14 carbon atoms, optionally substituted with at least one hetero atom; with at least one linear or branched aminoalkyl radical containing 1 to 4 carbon atoms, optionally substituted with at least one hetero atom; with at least one halogen atom;
• a fused or non-fused aromatic or diaromatic radical, optionally separated with an alkyl radical containing 1 to 4 carbon atoms, the aryl radical(s) optionally being substituted with at least one halogen atom or with at least one alkyl radical containing 1 to 10 carbon atoms optionally substituted and/or interrupted with at least one hetero atom and/or group bearing at least one hetero atom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound; 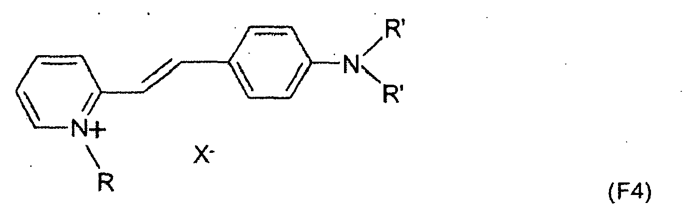
in which formula R represents a methyl or ethyl radical; R' represents a methyl radical and X⁻ represents an anion of the chloride, iodide, sulphate, methosulphate, acetate or perchlorate type.

26. Process according to Claims 21 to 25, **characterized in that** the hair is pigmented or artificially coloured.

27. Process according to any one of Claims 21 to 26, **characterized in that** the hair has a tone height of less than or equal to 4.

28. Process for dyeing with a lightening effect according to any one of Claims 21 to 27, consisting in performing the following steps:
a) the composition as defined in any one of Claims 21 to 25 is applied to the keratin fibres, for a time that is sufficient to develop the desired coloration and lightening,
b) the said fibres are optionally rinsed,
c) the said fibres are optionally washed with shampoo and rinsed,
d) the fibres are dried or are left to dry.

29. Process according to Claim 28, **characterized in that** it comprises a preliminary step that consists in separately storing, on the one hand, a composition as defined in one of Claims 21 to 25, and, on the other hand, a composition containing, in a cosmetically acceptable medium, at least one oxidizing agent, and then in mixing them together at the time of use, followed by applying this mixture to the fibres for a time that is sufficient to develop the desired coloration, after which the fibres are rinsed, optionally washed with shampoo, rinsed again and dried.

30. Process for colouring dark skin with a lightening effect, **characterized in that** a composition according to any one of Claims 1 to 13 is applied to the skin and the skin is then dried or is left to dry.

31. Multi-compartment device for dyeing and lightening human keratin fibres, comprising at least one compartment containing a composition according to one of Claims 1 to 13 and 15 to 18, and at least one other compartment containing a composition containing at least one oxidizing agent.

## Patentansprüche

1. Zusammensetzung, **dadurch gekennzeichnet, dass** sie in einem kosmetisch akzeptablen Medium mindestens einen in dem Medium löslichen, im Orarige-Bereich fluoreszierenden Farbstoff und mindestens ein Polyol der folgenden Formel (I) enthält, das eine Molmasse unter 500 g/mol aufweist und mehr aus 3 Kohlenstoffatome enthält: 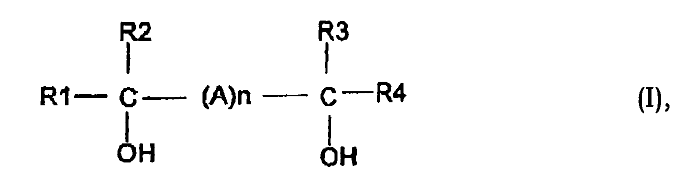 worin bedeuten:
A eine zweiwertige Gruppe einer geradkettigen oder verzweigten, gesättigten oder ungesättigten Kohlenwasserstoffgruppe, die gegebenenfalls durch 2 bis 10 Heteroatome unterbrochen wird und 1 bis 35 Kohlenstoffatome aufweist,
R1 bis R4 unabhängig voneinander ein Wasserstoffatom, C₁₋₂-Alkyl, Mono- oder Polyhydroxyalkyl mit 1 bis 6 Kohlenstoffatomen,
n 0 oder 1,
wobei die Zusammensetzung als fluoreszierenden Stoff kein 2-[2-(4-Dialkylamino)phenyl-ethenyl)-1-alkylpyridinium enthält, bei dem die Alkylgruppe des Pyridiniumrings eine Methylgruppe oder eine Ethylgruppe bedeutet und die Alkylgruppe des Benzolkerns eine Methylgruppe bedeutet und bei dem das Gegenion ein Halogenid ist.

2. Zusammensetzung nach Anspruch 1, **dadurch gekennzeichnet, dass** die Kohlenwasserstoffkette der zweiwertigen Gruppe A kein Heteroatom enthält.

3. Zusammensetzung nach einem der Ansprüche 1 oder 2, **dadurch gekennzeichnet, dass** die Gruppe A eine geradkettige oder verzweigte Alkylengruppe mit 1 bis 8 Kohlenstoffatomen bedeutet.

4. Zusammensetzung nach einem der Ansprüche 1 bis 3, **dadurch gekennzeichnet, dass** die Polyole der Formel (I) ausgewählt sind unter: 1,4-Butandiol, 1,5-Pentandiol, 1,6-Hexandiol, Neopentylglycol, Isoprenglycol, Hexylenglycol, 2,3-Dimethyl-1,5-pentandiol, 2-Methyl-1,3-propandiol, 3-Methyl-1,5-pentandiol, 3-Methyl-1,3,5-Pentantriol und 1,2,4-Butantriol, einzeln oder in Form von Gemischen.

5. Zusammensetzung nach einem der Ansprüche 1 bis 4, **dadurch gekennzeichnet, dass** das oder die Polyol(e) der Formel (I) 0,01 bis 30 Gew.-%, bevorzugt 0,1 bis 20 Gew.-% und insbesondere 0,5 bis 10 Gew.-% des Gesamtgewichts der Zusammensetzung ausmachen.

6. Zusammensetzung nach einem der vorhergehenden Ansprüche, **dadurch gekennzeichnet, dass** der fluoreszierende Farbstoff, der gegebenenfalls neutralisiert ist, in dem kosmetisch akzeptablen Medium in einer Menge von wenigstens 0,001 g/l, insbesondere mindestens 0,5 g/l, vorzugsweise mindestens 1 g/l und nach einer noch bevorzugteren Ausführungsform mindestens 5 g/l bei einer Temperatur von 15 bis 25 °C löslich ist.

7. Zusammensetzung nach einem der vorhergehenden Ansprüche, **dadurch gekennzeichnet, dass** der fluoreszierende Farbstoff zu einem maximalen Reflexionsgrad im Wellenlängenbereich von 500 bis 650 nm und vorzugsweise im Wellenlängenbereich von 550 bis 620 nm führt.

8. Zusammensetzung nach einem der vorhergehenden Ansprüche, **dadurch gekennzeichnet, dass** der fluoreszierende Farbstoff unter den fluoreszierenden Farbstoffen aus den folgenden Gruppen ausgewählt ist: Naphthalimiden; kationischen oder nicht kationischen Cumarinen; Xanthenodichinolizinen; Azaxanthenen; Naphtholactamen; Azlactonen; Oxazinen; Thiazinen; Dioxazinen; polykationischen fluoreszierenden Farbstoffen vom Azotyp, Azomethintyp oder Methintyp, wobei diese Farbstoffe einzeln oder im Gemisch vorliegen.

9. Zusammensetzung nach einem der vorhergehenden Ansprüche, **dadurch gekennzeichnet, dass** der fluoreszierende Farbstoff unter den Farbstoffen der folgenden Strukturen ausgewählt ist: 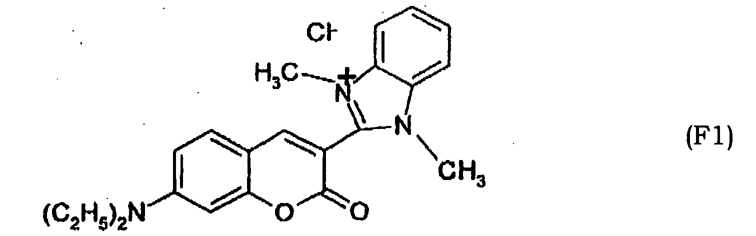 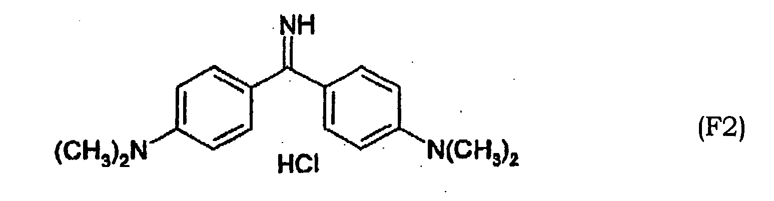 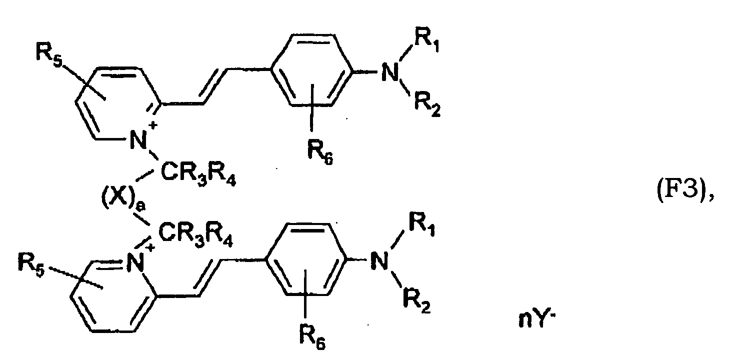 worin bedeuten:
die Gruppen R₁ und R₂, die gleich oder verschieden sind:
· ein Wasserstoffatom;
· eine geradkettige oder verzweigte Alkylgruppe mit 1 bis 10 Kohlenstoffatomen und vorzugsweise 1 bis 4 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· eine Aryl- oder Arylalkylgruppe, wobei die Arylgruppe 6 Kohlenstoffatome und die Alkylgruppe 1 bis 4 Kohlenstoffatome besitzt, wobei die Arylgruppe gegebenenfalls mit einer oder mehreren geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind und/oder mit mindestens einem Halogenatom substituiert sind;
· die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält;
die Gruppen R₃ und R₄, die identisch oder verschieden sind, ein Wasserstoffatom oder eine Alkylgruppe mit 1 bis 4 Kohlenstoffatomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
· eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/ oder einer Gruppe, die mindestens ein Heteroatom enthält, substituiert und/oder mit mindestens einem Halogenatom substituiert ist;
· eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
· eine aromatische Gruppe oder eine kondensierte oder nicht kondensierte, diaromatische Gruppe, die gegebenenfalls durch eine Alkylgruppe mit 1 bis 4 Kohlenstoffatomen separiert wird, wobei die Arylgruppe(n) gegebenenfalls mit mindestens einem Halogenatom oder mit mindestens einer Alkylgruppe mit 1 bis 10 Kohlenstoffatomen substituiert sind, die gegebenenfalls mit mindestens einem Heteroatom und/oder eine Gruppe, die mindestens ein Heteroatom enthält, substituiert und/oder durch mindestens ein Heteroatom und/oder eine Gruppe, die mindestens ein Heteroatom enthält, unterbrochen ist;
· eine Dicarbonylgruppe;
· wobei die Gruppe X eine oder mehrere kationische Ladungen umfassen kann;
wobei a 0 oder 1 beträgt;
die Gruppen Y⁻, die gleich oder verschieden sind, ein organisches oder anorganisches Anion;
wobei n eine ganze Zahl von mindestens 2 und höchstens der Anzahl der in der fluoreszierenden Verbindung vorliegenden kationischen Ladungen ist.

10. Zusammensetzung nach einem der vorhergehenden Ansprüche, **dadurch gekennzeichnet, dass** der oder die fluoreszierende(n) Farbstoff(e) in einer Konzentration von 0,01 bis 20 Gew.-%, insbesondere 0,05 bis 10 Gew.-% und vorzugsweise 0,1 bis 5 Gew.%, bezogen auf das Gesamtgewicht der Zusammensetzung, enthalten sind.

11. Zusammensetzung nach einem der vorhergehenden Ansprüche, **dadurch gekennzeichnet, dass** sie ferner mindestens einen zusätzlichen, nicht fluoreszierenden Direktfarbstoff vom nichtionischen, kationischen oder anionischen Typ enthält.

12. Zusammensetzung nach Anspruch 11, **dadurch gekennzeichnet, dass** die zusätzlichen, nicht fluoreszierenden Direktfarbstoffe unter den nitrierten Benzolfarbstoffen, Azofarbstoffen, Anthrachinon-Farbstoffen, Naphthochinon-Farbstoffen, Benzochinon-Farbstoffen, den von Triarylmethan abgeleiteten Farbstoffen oder deren Gemischen ausgewählt sind.

13. Zusammensetzung nach einem der Ansprüche 11 oder 12, **dadurch gekennzeichnet, dass** der oder die zusätzliche(n) Direktfarbstoff(e) 0,0005 bis 12 Gew.-% und vorzugsweise 0,005 bis 6 Gew.-% des Gesamtgewichts der Zusammensetzung ausmachen.

14. Zusammensetzung nach einem der vorhergehenden Ansprüche, **dadurch gekennzeichnet, dass** sie in Form eines aufhellenden und färbenden Haarwaschmittels vorliegt.

15. Zusammensetzung nach einem der vorhergehenden Ansprüche, **dadurch gekennzeichnet, dass** sie ferner mindestens eine Oxidationsbase enthält, die unter den p-Phenylendiaminen, Bisphenylalkylendiaminen, p-Aminophenolen, o-Aminophenolen und den heterocyclischen Basen sowie den Additionssalzen dieser Verbindungen mit einer Säure oder einem alkalischen Stoff ausgewählt ist.

16. Zusammensetzung nach Anspruch 15, **dadurch gekennzeichnet, dass** die Oxidationsbase(n) in Konzentrationen von 0,0005 bis 12 Gew.-% und insbesondere 0,005 bis 6 Gew.-%, bezogen auf das Gesamtgewicht der Zusammensetzung, enthalten sind.

17. Zusammensetzung nach einem der Ansprüche 15 oder 16, **dadurch gekennzeichnet, dass** sie ferner mindestens einen Kuppler enthält, der unter den m-Phenylendiaminen, m-Aminophenolen, m-Dihydroxybenzolen, heterocyclischen Kupplern oder den Additionssalzen dieser Verbindungen mit einer Säure oder einem alkalischen Stoff ausgewählt ist.

18. Zusammensetzung nach Anspruch 17, **dadurch gekennzeichnet, dass** der oder die Kuppler in Mengenanteilen von 0,0001 bis 10 Gew.-% und insbesondere 0,005 bis 5 Gew.-%, bezogen auf das Gesamtgewicht der Zusammensetzung, enthalten sind.

19. Zusammensetzung nach einem der vorhergehenden Ansprüchen, **dadurch gekennzeichnet, dass** sie mindestens ein Oxidationsmittel enthält.

20. Zusammensetzung nach Anspruch 19, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, und Enzymen, wie Peroxidasen und Oxidoreduktasen (2 oder 4 Elektronen), ausgewählt ist.

21. Verfahren zum Färben von Haaren mit aufhellender Wirkung, die einen Farbton von höchstens 6 aufweisen, unter Verwendung einer Zusammensetzung, die in einem kosmetisch akzeptablen Medium mindestens einen in dem Medium löslichen fluoreszierenden Farbstoff und mindestens ein Polyol der folgenden Formel (I) enthält, das eine Molmasse unter 500 g/mol aufweist und mehr aus 3 Kohlenstoffatome enthält: 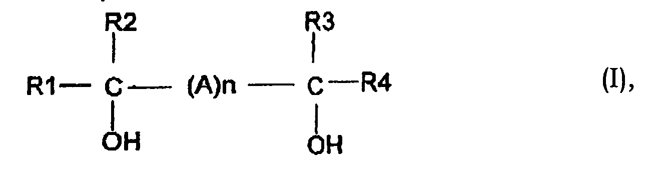 worin bedeuten:
A eine zweiwertige Gruppe einer geradkettigen oder verzweigten, gesättigten oder ungesättigten Kohlenwasserstoffgruppe, die gegebenenfalls durch 2 bis 10 Heteroatome unterbrochen wird und 1 bis 35 Kohlenstoffatome aufweist,
R1 bis R4 unabhängig voneinander ein Wasserstoffatom, C₁₋₂-Alkyl, C₁₋₆-Mono- oder C₁₋₆-Polyhydroxyalkyl,
n 0 oder 1.

22. Verfahren nach Anspruch 21, **dadurch gekennzeichnet, dass** der fluoreszierende Farbstoff ein Farbstoff im Orangebereich ist.

23. Verfahren nach einem der Ansprüche 21 oder 22, **dadurch gekennzeichnet, dass** der fluoreszierende Farbstoff zu einem maximalen Reflexionsgrad im Wellenlängenbereich von 500 bis 650 nm und vorzugsweise im Wellenlängenbereich von 550 bis 620 nm führt.

24. Verfahren nach einem der Ansprüche 21 bis 23, **dadurch gekennzeichnet, dass** der fluoreszierende Farbstoff unter den fluoreszierenden Farbstoffen aus den folgenden Gruppen ausgewählt ist: Naphthalimiden; kationischen oder nicht kationischen Cumarinen; Xanthenodichinolizinen (insbesondere Sulforhodaminen); Azaxanthenen; Naphtholactamen; Azlactonen; Oxazinen; Thiazinen; Dioxazinen; mono- oder polykationischen fluoreszierenden Farbstoffen vom Azotyp, Azomethintyp oder Methintyp, wobei diese Farbstoffe einzeln oder im Gemisch vorliegen.

25. Verfahren nach einem der Ansprüche 21 bis 24, **dadurch gekennzeichnet, dass** der fluoreszierende Farbstoff unter den Farbstoffen der folgenden Strukturen ausgewählt ist: 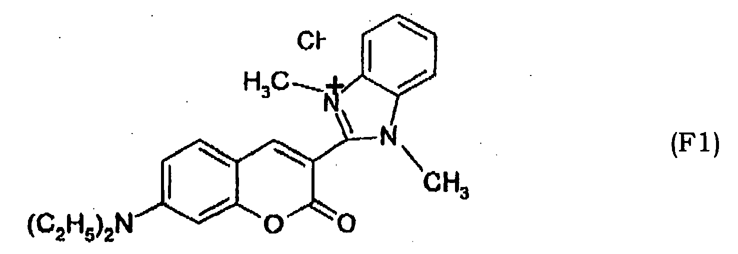 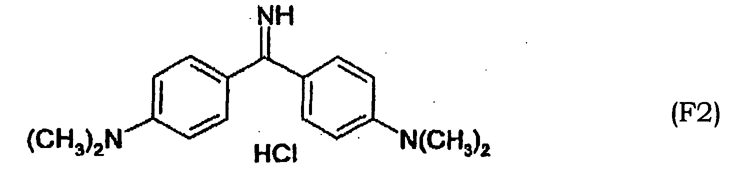 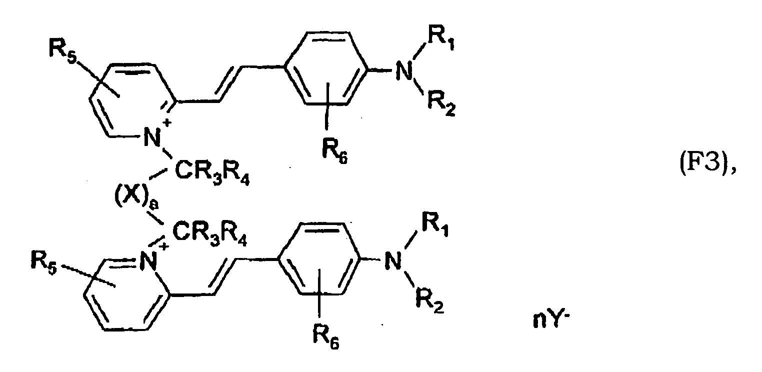 worin bedeuten:
die Gruppen R₁ und R₂, die gleich oder verschieden sind:
· ein Wasserstoffatom;
· eine geradkettige oder verzweigte Alkylgruppe mit 1 bis 10 Kohlenstoffatomen und vorzugsweise 1 bis 4 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· eine Aryl- oder Arylalkylgruppe, wobei die Arylgruppe 6 Kohlenstoffatome und die Alkylgruppe 1 bis 4 Kohlenstoffatome besitzt, wobei die Arylgruppe gegebenenfalls mit einer oder mehreren geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen sind und/oder mit mindestens einem Heteroatom und/oder einer Gruppe, die mindestens ein Heteroatom enthält, substituiert sind und/ oder mit mindestens einem Halogenatom substituiert sind;
· die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält;
die Gruppen R₃ und R₄, die identisch oder verschieden sind, ein Wasserstoffatom oder eine Alkylgruppe mit 1 bis 4 Kohlenstoffatomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
- eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert und/oder mit mindestens einem Halogenatom substituiert ist;
- eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
- eine aromatische Gruppe oder eine kondensierte oder nicht kondensierte, diaromatische Gruppe, die gegebenenfalls durch eine Alkylgruppe mit 1 bis 4 Kohlenstoffatomen separiert ist, wobei die Arylgruppe(n) gegebenenfalls mit mindestens einem Halogenatom oder mit mindestens einer Alkylgruppe mit 1 bis 10 Kohlenstoffatomen substituiert ist, die gegebenenfalls mit mindestens einem Heteroatom und/oder einer Gruppe, die mindestens ein Heteroatom enthält, substituiert und/oder durch mindestens ein Heteroatom und/oder eine Gruppe, die mindestens ein Heteroatom enthält, unterbrochen ist;
- eine Dicarbonylgruppe;
- wobei die Gruppe X eine oder mehrere kationische Ladungen
umfassen kann;
wobei a 0 oder 1 beträgt;
die Gruppen Y⁻, die gleich oder verschieden sind, ein organisches oder anorganisches Anion;
wobei n eine ganze Zahl von mindestens 2 und höchstens der Anzahl der in der fluoreszierenden Verbindung vorliegenden kationischen Ladungen ist; 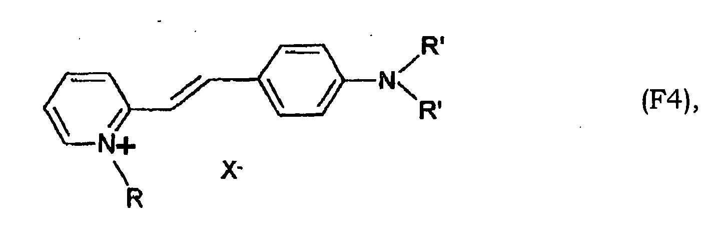 wobei in der Formel R eine Methylgruppe oder eine Ethylgruppe bedeutet; R' eine Methylgruppe ist, X⁻ ein Anion vom Typ Chlorid, Iodid, Sulfat, Methosulfat, Acetat oder Perchlorat bedeutet.

26. Verfahren nach Anspruch 21 bis 25, **dadurch gekennzeichnet, dass** die Haare pigmentierte oder künstlich gefärbte Keratinfasern sind.

27. Verfahren nach einem der Ansprüche 21 bis 26, **dadurch gekennzeichnet, dass** die Haare einen Farbton von 4 oder darunter aufweisen.

28. Verfahren zum Färben nach einem der Ansprüche 21 bis 27 mit aufhellender Wirkung, das darin besteht, dass die folgenden Schritte durchgeführt werden:
a) auf die Keratinfasern wird eine Zusammensetzung nach einem der Ansprüche 21 bis 25 während einer Zeitspanne aufgebracht, die ausreichend ist, um die gewünschte Färbung und Aufhellung zu erzielen,
b) die Fasern werden gegebenenfalls gespült,
c) die Fasern werden gegebenenfalls mit Haarwaschmittel gewaschen und gespült,
d) die Fasern werden getrocknet oder trocknen gelassen.

29. Verfahren nach Anspruch 28, **dadurch gekennzeichnet, dass** es einen vorbereiteten Schritt umfasst, der darin besteht, einerseits eine Zusammensetzung nach einem der Ansprüche 21 bis 25 und andererseits eine Zusammensetzung, die in einem kosmetisch akzeptablen Medium mindestens ein Oxidationsmittel enthält, getrennt voneinander aufzubewahren und sie bei der Anwendung, bevor das Gemisch während einer Zeitspanne, die ausreichend ist, um die gewünschte Färbung zu bilden, auf die Fasern aufgebracht wird, zu vermischen, worauf gespült, gegebenenfalls mit Haarwaschmittel gewaschen, nochmals gespült und getrocknet wird.

30. Verfahren zum Färben von dunkler Haut mit aufhellender Wirkung, **dadurch gekennzeichnet, dass** auf die Haut eine Zusammensetzung nach einem der Ansprüche 1 bis 13 aufgetragen und die Haut anschließend getrocknet oder trocknen gelassen wird.

31. Vorrichtung mit mehreren Abteilungen zum Färben und Bleichen von menschlichen Keratinfasern mit mindestens einer Abteilung, die eine Zusammensetzung nach einem der Ansprüche 1 bis 13 und 15 bis 18 enthält, und mindestens einer weiteren Abteilung, die eine Zusammensetzung enthält, die mindestens ein Oxidationsmittel umfasst.
